# EUROPEAN PATENT APPLICATION

(11) **EP 2 308 864 A1**
(43) Date of publication of application: **13.04.2011**
(21) Application number: 10186667.1
(22) Date of filing: 16.02.2007
(51) Int. Cl.: C07D 333/20, A61K 9/50, A61K 31/381

(54) **Pharmaceutical formulation of duloxetine hydrochloride**

(30) Priority: 17.02.2006 EP 06003244
(62) Divisional of application: 07703509.5
(71) Applicant: KRKA, tovarna zdravil, d.d., Novo mesto, 8501 Novo mesto (SI)
(72) Inventor: Stimac, Anton, 1000, Ljubljana (SI); Zajc, Natalija, 8340, Crnomelj (SI); Vajs, Anamarija, 8000, Novo Mesto (SI); Jakse, Renata, 8220, Smarjeske Toplice (SI); Zupet, Rok, 1000, Ljubljana (SI)
(74) Representative: HOFFMANN EITLE

(57) **Abstract**

The present invention relates to polymorphic forms of duloxetine hydrochloride, especially (duloxetine HCl), which are characterized by an X-ray diffraction pattern with peaks (2θ):9.6, 13.9, 18.0, 18.8, 19.2, 20.8, 27.4, 27.9 (designated form A) or an X-ray diffraction pattern with characteristic peaks (2θ): 12.0, 14.8, 19.8, 21.3, 21.6, 22.1, 22.4, 23.1, 24.1 (designated form T). The present invention also pertains to processes for the preparation of form A and form T, which involves dissolving duloxetine hydrochloride in particular solvents and cooling the solution to obtain crystals, that are dried.

## Description

### Field of the Invention

The present invention relates to polymorphic forms of duloxetine hydrochloride, especially (duloxetine HCI), which are characterized by an X-ray diffraction pattern with peaks (2θ):9.6, 13.9, 18.0, 18.8, 19.2, 20.8, 27.4, 27.9 (designated form A) or an X-ray diffraction pattern with characteristic peaks (2θ): 12.0, 14.8, 19.8, 21.3, 21.6, 22.1, 22.4, 23.1, 24.1 (designated form T). The present invention also pertains to processes for the preparation of form A and form T, which involves dissolving duloxetine hydrochloride in particular solvents and cooling the solution to obtain crystals, that are dried.

### Background of the Invention

Duloxetine, (*S*)-*N*-methyl-3-(1-naphtalenyloxy)-3-(2-thienyl)propanamine, is a dual serotonin and norepinephrine re-uptake inhibitor. Duloxetine has particular therapeutic utility as an anti-depressant. It may be prepared in form of salts, such as the oxalate salt, maleate salt, hydrochloride salt, di-p-toluyl tartarate salt, or any other pharmaceutically suitable salt, as e.g. disclosed in WO 2005/108386, page 9.

Duloxetine, and methods for its synthesis is described e.g. in US-P-5,023,269 and US-P-4,956,388, and also in Tetrahedron Letters (1990) 31(49), 7101-7104. Seven different routes of synthesis have also been reported in Drugs of the Future (2000) 25(9), 907-916. They all involve either a resolution of a key intermediate or a stereospecific reduction of a keto group to the alcohol.

In US-P-5,023,269 and US-P-4,956,388 preparation of duloxetine maleate and oxalate salts is disclosed. In both, US-P-4,956,388 as well as in US-P-5,023,269, the preparation of the racemic oxalate salt of duloxetine with melting intervals of 136-138.5 °C and 148-150 °C, from a mixture of ethyl acetate and methanol is disclosed, in examples 2 and 9, respectively. Example 14 discloses the preparation of (+)-duloxetine maleate with a melting interval of 118-122 °C, while example 38 discloses the preparation of (+)-duloxetine oxalate with a melting interval of 133-134°C.

In Tetrahedron Letters (1990) 31 (49), 7101-7104, the preparation of duloxetine maleate and duloxetine oxalate from ethyl acetate is described.

In Journal of Labeled Compounds and Radiopharmaceuticals (1995) 36(3), 213-223, duloxetine HCl (form A) is described to have been prepared by dissolving duloxetine free base in ethyl acetate and treating the solution dropwise with 0.44 M solution of hydrogen chloride in ethyl acetate, and subsequently diluting it with ether. The duloxetine HCl obtained was recrystallized from ethanol/ether.

In WO 2005/019199 the preparation of amorphous duloxetine HCl by dissolving it in an alcohol, a ketone or an ester solvent and removing the solvent by means of vacuum drying or spray drying is made known.

In WO 2005/108386 polymorphic forms of duloxetine free base are described. On the one hand they may be prepared from duloxetine salts and on the other they may also be applied for the preparation of duloxetine salts. In example 4 the preparation of duloxetine HCl from duloxetine free base (Form A) from acetone is described, using a 20% isopropanolic solution of HCl. Example 5 discloses the use of duloxetine free base form B.

In EP 0 650 965 the preparation of duloxetine HCl from ethyl acetate is described.

The present invention relates to processes for the preparation of solid state duloxetine HCl and their physical properties. These properties may be influenced by controlling the conditions under which duloxetine HCl is obtained in solid form. Solid state physical properties include, for example, the flowability of the milled solid, which is known to affect the ease with which the material is handled during processing into a pharmaceutical product. When particles of the powdered compound do not flow past each other easily, a formulation specialist must take that fact into account when developing a tablet or capsule formulation, which may necessitate the use of glidants such as colloidal silicon dioxide, talc, starch or tribasic calcium phosphate. Another important solid state property of a pharmaceutical compound is its rate of dissolution in aqueous fluid. The rate of dissolution of an active ingredient in a patient's stomach fluid can have therapeutic consequences since it imposes an upper limit on the rate at which an orally-administered active ingredient can reach the patient's bloodstream. The solid state form of a compound may also affect its behaviour on compaction and its storage stability.

These practical physical characteristics are influenced by the conformation and orientation of molecules in the unit cell, which defines a particular polymorphic form of a substance. The polymorphic form may give rise to thermal behaviour which is different from that of the amorphous material or another polymorphic form. Thermal behaviour is measured in the laboratory by such techniques as capillary melting point, thermogravimetric analysis (TGA) and differential scanning calorimetry (DSC) and can be used to distinguish some polymorphic forms from others. A particular polymorphic form may also give rise to distinct spectroscopic properties that may be detectable by powder X-ray crystallography, solid state ¹³C NMR spectrometry and infrared spectrometry.

Processes for the preparation of duloxetine HCl already disclosed in prior art either disclose the use of solvent mixtures, such as ethanol/diethyl ether, which are not optimal to be applied in industrial scale, e.g. due to explosivity of their vapours in mixture with air, or due to their toxicity, or do not lead to optimal yields or purities required for pharmaceutically active compounds.

There are now provided by the present invention, processes for the preparation of solid duloxetine HCl, suitable for the preparation of solid duloxetine HCl on industrial scale.

The invention provides a process for the preparation of a new polymorphic form of duloxetine HCl, herein designated form T, which polymorphic form represents a further embodiment of the present invention. In addition, the invention provides new processes for the preparation of polymorphic form of duloxetine HCl, herein designated form A. As used herein, the term "duloxetine" preferably designates (*S*)-duloxetine.

### Summary of the Invention

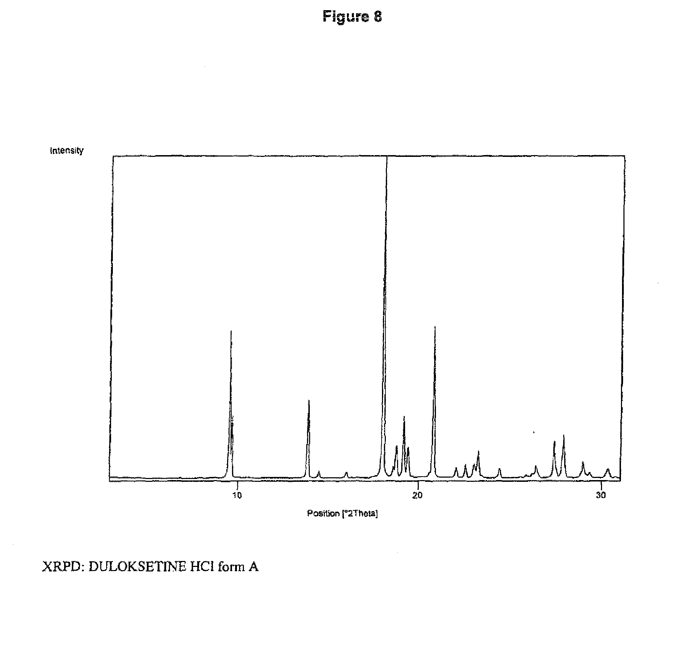

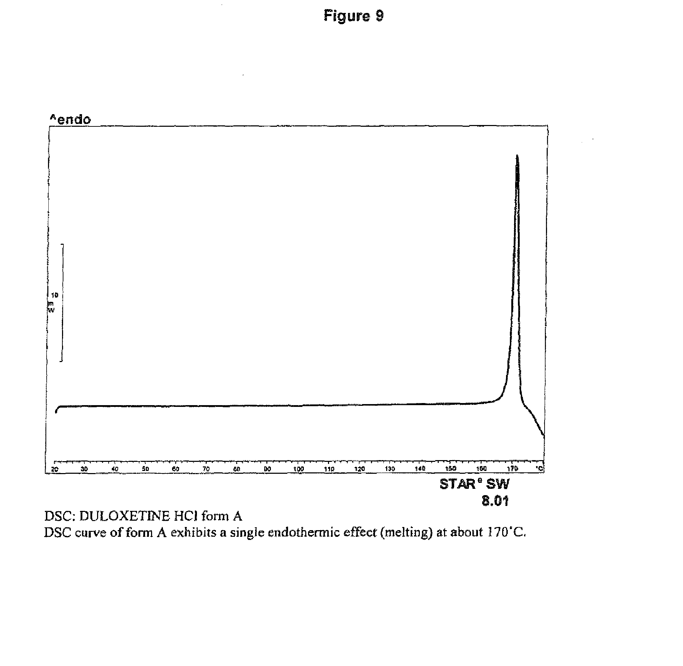

Described herein are processes for the preparation of crystalline duloxetine HCl, form A, having an X-ray diffraction pattern, or substantially the same X-ray diffraction pattern, as shown in Figure 8. More particularly, crystalline duloxetine HCl form A according to the present invention can be characterized as having an X-ray diffraction pattern with characteristic peaks (2θ):9.6, 13.9, 18.0, 18.8, 19.2, 20.8, 27.4, 27.9, and a single endothermic effect of about 170°C in the DSC curve as shown in Figure 9.

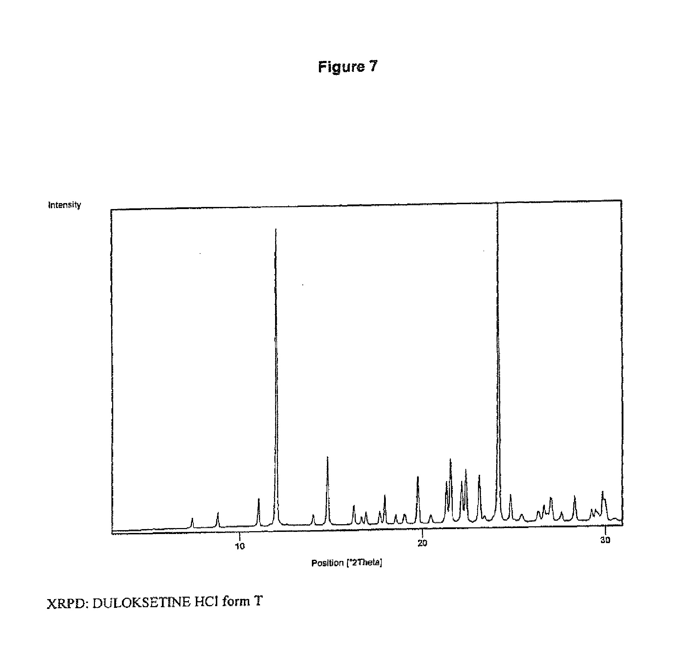

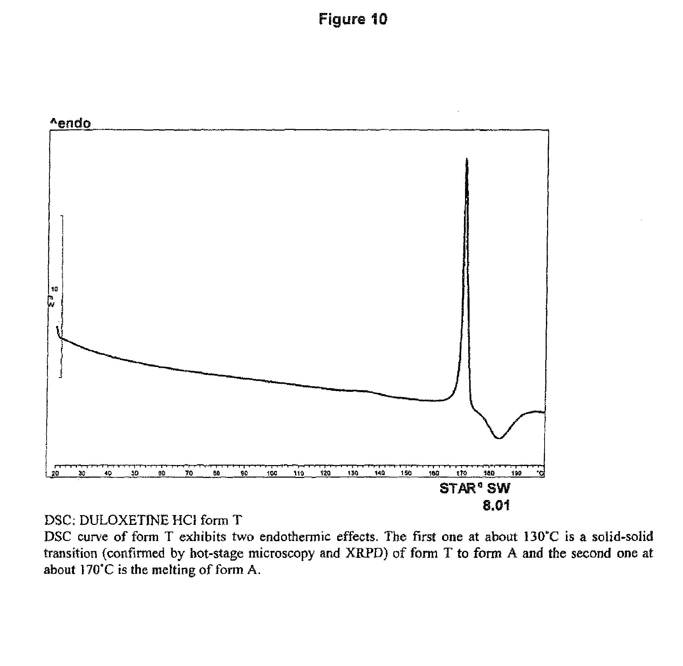

According to an aspect of the invention, there is provided crystalline duloxetine HCl, Form T, having an X-ray diffraction pattern, or substantially the same X-ray diffraction pattern, as shown in Figure 7. More particularly, crystalline duloxetine HCl form T according to the present invention can be characterized as having an X-ray diffraction pattern with characteristic peaks (2θ): 12.0, 14.8, 19.8, 21.3, 21.6, 22.1, 22.4, 23.1, 24.1, and two endothermic effects as shown in Figure 10. The first endothermic effect of about 130°C represents a solid-solid transition of form T to form A and the second one at about 170°C represents the melting point of form A.

### Brief Description of the Drawings

Fig. 1 represents an FT-IR spectrum of duloxetine HCl form A.
Fig. 2 represents a characterized FT-IR spectrum of duloxetine HCl form A.
Fig. 3 represents an FT-IR spectrum of duloxetine HCl form T.
Fig. 4 represents a characterized FT-IR spectrum of duloxetine HCl form T.
Fig. 5 represents a Raman spectrum of duloxetine HCl form A.
Fig. 6 represents a Raman spectrum of duloxetine HCl form T.
Fig. 7 discloses an X-ray powder diffractogram of duloxetine HCl form T.
Fig. 8 discloses an X-ray powder diffractogram of duloxetine HCl form A.
Fig. 9 shows a DSC curve of duloxetine HCl form A. DSC curve of form A exhibits a single endothermic effect (melting) at about 170°C.
Fig. 10 shows a DSC curve of duloxetine HCI form T. DSC curve of form T exhibits two endothermic effects. The first one at about 130°C is a solid-solid transition (confirmed by hot-stage microscopy and XRPD) of form T to form A and the second one at about 170°C is the melting of form A.

### Detailed Description of the Invention

As used herein, the term "drying" generally refers to a removal of solvent until duloxetine HCI contains less than about 6000 ppm residual solvents. Drying may be achieved e.g. via application of heat, preferably carried out under ambient or reduced pressure or via contacting duloxetine HCl with humid air in a fluidized bed drier, wherein the atmosphere in the fluidized bed drier is at least 15% humidity.

The term "reduced pressure" refers to a pressure below one atmosphere, more preferably below about 100 mmHg.

As used herein, the term "precipitation" refers to the formation of a suspension of small solid particles in a mixture.

As used herein, the term "crystallization" refers to a process for forming crystals from a liquid or gas.

The present description highlights processes for the preparation of crystalline form A of duloxetine HCl, which form A is characterized by an X-ray diffraction pattern with peaks at 9.6, 13.9, 18.0, 18.8, 19.2, 20.8, 27.4, 27.9 ±0.2 degrees 2-theta. Additional XRPD peaks are listed in Table I.

Duloxetine HCl form A may be crystallized out of a solution of duloxetine HCl in water, alcohols, such as, methanol, ethanol, 1-propanol, 2-propanol, 1-butanol, isobutanol, tert.-buta - nol, 2-methoxyethanot, 2,2,2-trifluoroethanol; or acetonitrile, nitromethane, 1,2-dimethoxyethane; or esters, such as methyl acetate, ethyl formate; or ketones, such as e.g. acetone, 2-butanone; or mixtures thereof, or mixtures with water. A solution of duloxetine HCl is prepared in these solvents, preferably by warming up, e.g. to a temperature of from about 45°C to about 120 °C, and most preferably under reflux temperature of the solvent applied. The solution is cooled to induce crystallization. Preferably, the solution is cooled to a temperature of from -20 °C to about 30 °C, more preferably from about -10 °C to about 20 °C. The resulting crystals may then be recovered by techniques well known in the art, such as filtration, centrifugation, decanting etc. While duloxetine HCl is being recovered from these solvents and before it is dried, the formation of solvates may occur.

In an aspect, the present invention provides for crystalline form T of duloxetine HCl. Duloxetine HCl form T is characterized by an X-ray diffraction pattern with peaks at 12.0, 14,8, 19.8, 21.3, 21.6, 22.1, 22.4, 23.1, 24.1±0.2 degrees 2-theta. Additional XRPD peaks are listed in Table II.

Duloxetine HCl form T may be crystallized out of a solution of duloxetine HCl in 1,4-dioxane, or its mixtures with water or other solvents, such as methanol. A solution of duloxetine HCl is prepared in this solvent or its mixture with water and/or other solvent, preferably by warming up to a temperature of from about 85°C to about 105°C, most preferably to reflux temperature of the solvent used. The solution is cooled to induce crystallization. Preferably the solution is cooled to a temperature of from -20°C to about 30°C, more preferably from about -10°C to about 20°. The resulting crystals then may be recovered by techniques well known in the art, such as filtration, centrifugation, decanting etc. While duloxetine HCl is being recovered from 1,4-dioxane or its mixtures with other solvents, and before it is dried, the formation of solvates may occur.

The crystals may then be dried. Drying may be carried out under ambient or reduced pressure. Preferably, drying is carried out at a temperature of from 20°C to about 100 °C, more preferably in combination with a pressure of less than about 100 mbar. Approximately a few hours of drying, e,g. about 1 to about 72 hours, depending on the conditions, may be sufficient. Duloxetine HCl according to the invention may also be contacted with humid air in a fluidized bed drier, wherein the atmosphere in the fluidized bed drier is at least 15% humidity, until duloxetine HCl contains less than about 6000 ppm residual solvents.

The X-ray diffraction peaks of the two different forms of duloxetine HCl are summarized in the following tables ±0.2 degrees 2-theta. X-ray powder diffraction patterns were obtained by Phillips PW3040/60 X'Pert PRO powder diffractometer; CuKa radiation 1,541874Å, 3°<2θ<31°.

A typical X-ray diffraction pattern of crystalline form A of duloxetine HCl is given in the following Table I by listing 2-theta degrees together with the corresponding intensities. In the table "s" designates a strong relative intensity from 100 to 40 %, "m" designates a medium relative intensity from 40 to 10 % and "w" designates a weak relative intensity less than 10%.

**Table I:**

| | **Intensity** |
|---|---|
| **2Θ** | |
| 9.6 | m |
| 13.9 | m |
| 18.0 | s |
| 18.8 | m |
| 19.2 | m |
| 19.5 | w |
| 20.8 | s |
| 23.3 | w |
| 27.4 | m |
| 27.9 | m |

Further, the crystalline form A of duloxetine HCl form A according to the invention is characterized by the DSC curve shown in Fig. 9 with the onset temperature at about 170°C.

All DSC scans were recorded on DSC 822e Mettler Toledo scanning calorimeter. Samples of approx. 3 mg were scanned between 20 °C and 200 °C at a heating rate of 10 °C/min under nitrogen atmosphere.

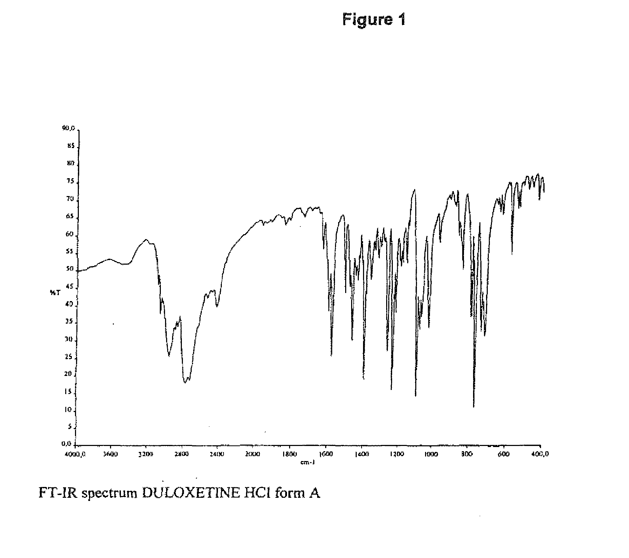

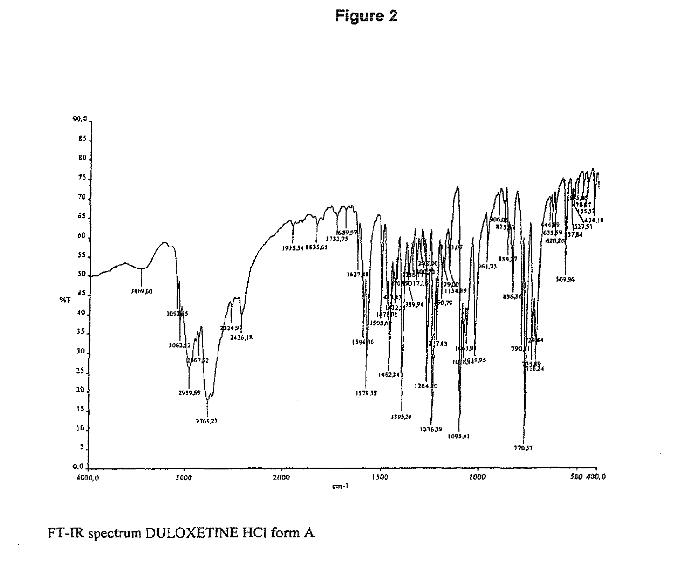

Typical FT-IR spectra of KBr discs for duloxetine. HCl form A as shown in Figures 1 and 2 were recorded over a wave number range of 4000 - 400 cm⁻¹ on Perkin Elmer FT-IR Spectrometer Spectrum GX at a resolution of 4 cm⁻¹. The most characteristic infrared absorption bands of form A are at: 3093, 2525, 1444, 1432, 1360, 1336, 1179, 1095, 1078, 1020, 962, 836, 771, 736, 716, 614 cm⁻¹.

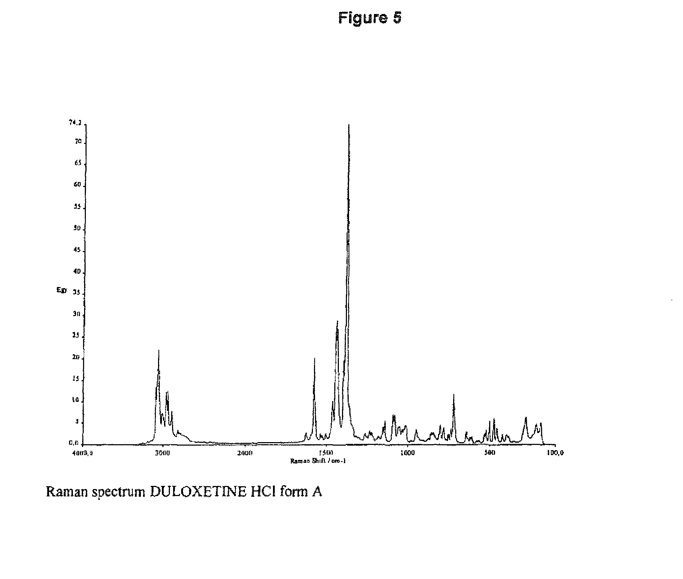

Raman spectra were obtained by Perkin Elmer FT-Raman Spectrometer Spectrum GX over a wave number range 4000-100 cm⁻¹ at a resolution of 4 cm⁻¹, Raman laser power: 500 mW. A typical Raman spectrum of form A of duloxetine HCI is shown in Figure 5. The most characteristic peaks in Raman spectrum of form A are: 3093, 3063, 3020, 2943, 2899, 1580, 1443, 506 cm⁻¹.

A typical X-ray diffraction pattern of crystalline form T of duloxetine HCl is given in the following Table II by listing 2-theta degrees together with the corresponding intensities ±0.2 degrees 2-theta. In the table "s" designates a strong relative intensity from 100 to 40 %, "m" designates a medium relative intensity from 40 to 10 % and "w" designates a weak relative intensity less than 10%.

**Table II:**

| | **Intensity** |
|---|---|
| **2Θ** | |
| 8.8 | w |
| 11.0 | w |
| 12.0 | s |
| 14.8 | m |
| 16.2 | w |
| 19.8 | m |
| 21.3 | m |
| 21.6 | m |
| 22.1 | m |
| 22.4 | m |
| 23.1 | m |
| 24.1 | s |
| 24.8 | w |
| 26.6 | w |
| 26.9 | w |
| 28.3 | w |
| 29.8 | w |
| 30.0 | w |

Further, the crystalline form T of duloxetine HCl according to the invention is characterized by the DSC curve shown in Figure 10. DSC curve of form T exhibits two endothermic effects. The first one at about 130°C is a solid-solid transition (confirmed by hot-stage microscopy and XRPD) of form T to form A and the second one at about 170°C is the melting of form A. DSC scans were recorded on DSC 822e Mettler Toledo scanning calorimeter. Samples of approx. 3 mg were scanned between 20°C and 200°C at heating rate of 10°C/min under nitrogen atmosphere.

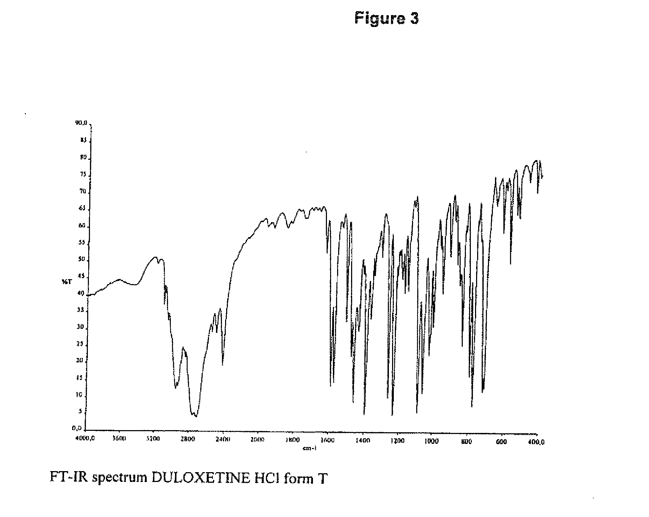

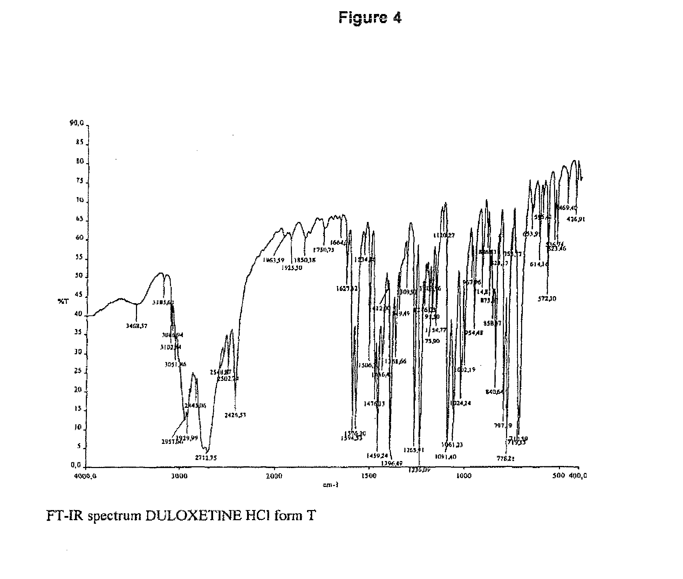

Typical FT-IR spectra of KBr discs as shown in Figures 3 and 4 were recorded over a wave number range of 4000 - 400 cm⁻¹ on Perkin Elmer FT-IR Spectrometer Spectrum GX at a resolution of 4 cm⁻¹. The most characteristic infrared absorption bands of form T are at: 3103, 2549, 2503, 1459, 1436, 1369, 1176, 1091, 1024, 1002, 954, 915, 841, 797, 778 cm⁻¹.

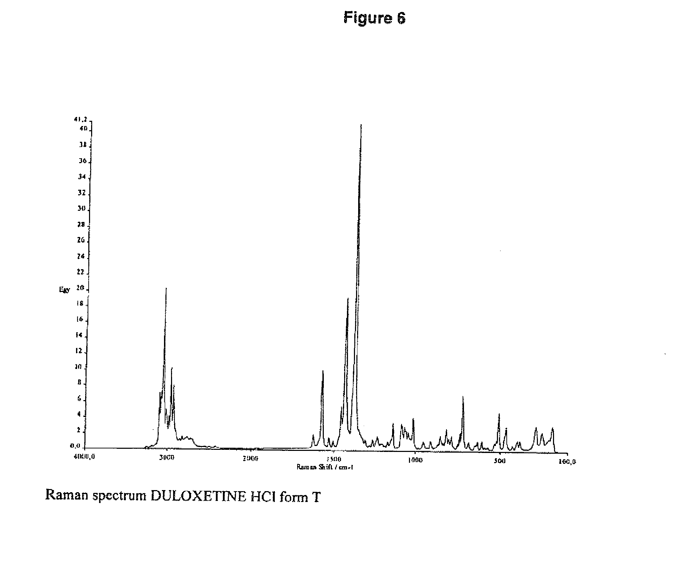

Raman spectra were obtained by Perkin Elmer FT-Raman Spectrometer Spectrum GX over a wave number range 4000-100 cm⁻¹ at a resolution of 4 cm⁻¹, Raman laser power: 500 mW. A typical Raman spectrum of form T of duloxetine HCl is shown in Figure 6. The most characteristic peaks in Raman spectrum of form T are: 3104, 3059, 3024, 2989, 2331, 1577, 5 12, 469 cm⁻¹.

The starting material used for the processes of the present invention may be any crystalline or amorphous form of duloxetine HCl, including any solvates and hydrates. With processes where duloxetine HCl goes into solution, the form of the starting material is of minimal relevance since any solid state structure is lost in solution.

Amorphous form of duloxetine HCl or its pharmaceutically acceptable salts and/or pharmaceutical excipients may also be obtained by milling the crystalline material. In this process the mechanical force on the particle surface leads to particle size reduction, however it may also release structure changes of the material. For this purpose air jet mill, ball mill or hammer mill are commonly used as milling equipment.

The basic principle of treatment in air jet mill is collision and attrition between particles suspended within the high velocity air stream, which introduces the power to the milling chamber. In the ball mill the particles are fractured by impact of grinding media (balls, cubes, cylinders, etc.) that can occupy up to half of the mill chamber volume.

The grinding media is falling from the elevated position due to rotation of the chamber. Friction is also present among all elements, contributing significantly to the attrition and consequently to amorphous nature of the material being milled. One of the most widely used mills in the pharmaceutical industry is the hammer mill. In such equipment particles are exposed to impact of rapidly rotating hammers. During milling material additionally hit the perforated screen that is placed over the chamber outlet.

Pharmaceutical formulations of the present invention contain crystalline duloxetine. HCl, such as one of those disclosed herein, or duloxetine HCl purely amorphous, optionally in mixture with other form(s) of duloxetine HCl. The duloxetine HCl prepared by the processes of the present invention are ideal for pharmaceutical formulation. In addition to the active ingredient(s), the pharmaceutical compositions of the present invention may contain one or more excipients. Excipients are added to the composition for a variety of purposes.

A preferred core for the gastroresistent pellet is prepared by applying a duloxetine-containing layer to an inert bead. Such inert beads are conventionally used in pharmaceutical science, and are readily purchased in all industrial countries. The most preferred bead is one prepared from starch and sucrose, for use in confectionery as well as in pharmaceutical manufacturing. However, beads of any pharmaceutically acceptable excipient may be used, including, for example, microcrystalline cellulose, vegetable gums, waxes, and the like. The primary characteristic of the inert bead is to be inert, with regard both to duloxetine and the other excipients in the pellet and with regard to the patient who will ultimately ingest the pellet.

The size of the beads depends, of course, on the desired size of the pellet to be manufactured. In general, pellets can be as small as 0.1 mm, or as large as 2 mm. Preferred beads are from about 0.3 to about 0.8 mm, in order to provide finished pellets in the desired preferred size range of from about 0.5 to about 1.5 mm in diameter.

It is always preferred for the beads to be of a reasonably narrow particle size distribution, in order to improve the uniformity of the various coatings to be added and the homogeneity of the final product. For example, the beads may be specified as being of particle size ranges such as from 18 to 20 U.S. mesh, from 20 to 25 U.S. mesh, or from 25 to 35 U.S. mesh to obtain acceptable size distributions of various absolute sizes.

The amount of beads to be used obviously depends on the weights and thicknesses of the added layers; in general, the beads comprise from about 15 to about 70 percent of the product. More preferably, the charge of beads represents from about 35 to about 65 percent of the product.

When manufacture of the pellet begins with inert beads, the duloxetine is coated on the beads to yield a final drug concentration of about I to about 15 percent of the product, in general. The amount of duloxetine, of course, depends on the desired dose of the drug and the quantity of pellets which it is desired to administer. The dose of duloxetine is in the range of 1-50 mg, more usually 5-20 mg, and the usual amount of pellets is that amount which is conveniently held in gelatine capsules. Comparison of the volume of gelatine capsules and the desired doses leads the pharmacist to the concentration range of from about 1% to about 15% of duloxetine in the present product.

Attention should be given to the particle size of duloxetine. The compound may precipitate in needle-like crystals which may be quite large. Coating beads with duloxetine in a large needle-like form may be difficult, and it may be advisable to mill or otherwise reduce the particle size of the duloxetine to less than about 120 µm, preferably less then about 50 µm before using it in the pharmaceutical formulation or process for its preparation. Duloxetine HCl particles as small as 12 microns were obtained using Hosokawa Alpine 100 UPZ hammer mill.

A convenient manner of coating the beads with duloxetine is the "powder coating" process where the beads are moistened with a sticky liquid or binder, duloxetine is added as a powder, and the mixture is dried. Such a process is regularly carried out in the practice of industrial pharmacy, and suitable equipment is in daily use.

Such equipment is, in fact, used in several steps of the present process, and it will, accordingly, be discussed in detail here. Historically, this process has been conducted in conventional coating pans similar to those employed in sugar coating processes. This process can be used to prepare pellets, but this equipment has less efficient air flow and drying capabilities which limits application rates and can result in longer processing times in order to minimize agglomerations.

Alternatively, the present product could be made in fluidized bed equipment (using a rotary processor), or in rotating plate equipment such as the Freund CF-Granulator (Vector Corporation, Marion, Iowa). The rotating plate equipment typically consists of a cylinder, the bottom of which is a rotatable plate. Motion of the mass of particles to be coated is provided by friction of the mass between the stationary wall of the cylinder and the rotating bottom of it. Means can be provided to apply warm air to dry the mass, and liquids can be sprayed on the mass and balanced against the drying rate as in the fluidized bed case.

When a powder coating is to be applied, the mass of pellets, in the present case, is maintained in a sticky state, and the powder to be adhered thereto, duloxetine, is continuously or periodically added and adheres to the sticky pellets. When all of the duloxetine has been applied, the spray is stopped and the mass is allowed to dry in the air stream. It may be appropriate or convenient to add some inert powders to the duloxetine.

Additional solids may be added to the layer with duloxetine. These solids may be added to facilitate the coating process as needed to aid flow, reduce static charge, aid bulk build-up and form a smooth surface. Inert substances such as talc, kaolin, and titanium dioxide, lubricants such as magnesium stearate, finely divided silicon dioxide, crospovidone, and beta -lactose may be used. The amounts of such substances are in the range from about a few tenths of 1% of the product, up to about 20% of the product. Such solids should be of fine particle size, less than 50 µm, to produce a smooth surface.

Duloxetine may be made to adhere to the beads by spraying a pharmaceutical excipient which is sticky and adherent when wet, and dries to a strong, coherent film. Pharmaceutical scientists are aware of and conventionally use many such substances, most of them polymers. Preferred polymers include hydroxypropylmethylcellulose, hydroxypropylcellulose and polyvinylpyrrolidone. Additional such substances include methylcellulose, carboxymethylcellulose, acacia and gelatine, for example. The amount of the adhering excipient is in the range from about a few tenths of 1% to about 5% of the product, and depends in large part on the amount of duloxetine to be adhered to the bead.

Duloxetine may also be built up on the beads by spraying a slurry comprising duloxetine suspended in a solution of the excipients of the duloxetine layer, dissolved or suspended in sufficient water to make the slurry sprayable. Such a slurry may be milled through a machine adapted for grinding suspensions in order to reduce the particle size of duloxetine. Grinding in suspension form is desirable because it avoids dust generation and containment problems which arise in grinding dry powder drugs. A preferred method for applying this suspension is in the classic pharmaceutical fluidized bed coating device, such as the Wurster column, which consists simply of a vertical cylinder with an air-permeable bottom and an upward spraying nozzle close above the bottom, or a downward-spraying nozzle mounted above the product mass. The cylinder is charged with particles to be coated, sufficient volume of air is drawn through the bottom of the cylinder to suspend the mass of particles, and the liquid to be applied is sprayed onto the mass. The temperature of the fluidizing air is balanced against the spray rate to maintain the mass of pellets or tablets at the desired level of moisture and stickiness while the coating is built up.

On the other hand, the core may comprise a monolithic particle in which the duloxetine is incorporated. Such cores may be prepared by the granulation techniques which are wide spread in pharmaceutical science, particularly in the preparation of granular material for compressed tablets. The particle size of the cores is too small for preparation by compression techniques, but the cores may be prepared by mixing the duloxetine into a mass of pharmaceutical excipients, moistening the mass with water or a solvent, drying, and breaking the mass into sized particles in the same size range as described above for the inert beads. This may be accomplished via the process of extrusion and marumerization.

One or more enteric coated layers are applied onto the core material or onto the core material covered with separating layer by using a suitable coating technique. The enteric coating layer material may be dispersed or dissolved in either water or in suitable organic solvents. As enteric coating layer polymers one or more, separately or in combination, of the following can be used: solutions or dispersions of methacrylic acid copolymers, cellulose acetate phthalate, hydroxypropyl methylcellulose phthalate, hydroxypropyl methylcellulose acetate succinate, polyvinyl acetate phthalate, cellulose acetate trimellitate, carboxymethylethyl-cellulose or other suitable enteric coating layer polymers.

The enteric coating layers contain pharmaceutically acceptable plasticizers to obtain the desired mechanical properties, such as flexibility and hardness of the enteric coating layers. Such plasticizers are for the instance, but not restricted to, triacetin, citric acid esters, phthalic acid esters, dibutyl sebacate, dimethyl polysiloxan, cetyl alcohol, stearyl alcohol, polyethylene glycols, propylenglycol, polysorbates or other plasticizers. Additives such as dispersants, colorants, pigments, polymers, anti-tacking agent and antifoaming agents may also be included into the enteric coating layer.

The smoothing function of the separating layer is purely mechanical, the objective of which is to improve the coverage of the enteric layer and to avoid thin spots in it, caused by bumps and irregularities on the core. Accordingly, the more smooth and free of irregularities the core can be made, the less material is needed in the separating layer, and the need for the smoothing characteristic of the separating layer may be avoided entirely when the duloxetine is of extremely fine particle size and the core is made as close as possible to truly spherical.

It has been found that, when a pharmaceutically acceptable sugar is added to the separating layer, the pellet's resistance to acid conditions is markedly and surprisingly increased. Accordingly, such a sugar may be included in the separating layer applied to the beads, either as a powdered mixture, or dissolved as part of the sprayed-on liquid. A sugar-containing separating layer can reduce the quantity of enteric polymer required to obtain a given level of acid resistance. It therefore considerably reduces the expense of the present formulated product. Use of less enteric polymer reduces both the materials cost and processing time, and also reduces the amount of polymer available to react with duloxetine. The inhibition of any core/enteric layer interaction is mechanical. The separating layer physically keeps the components in the core and enteric layers from coming into direct contact with each other. In some cases, the separating layer can also act as a diffusional barrier to migrating core or enteric layer components dissolved in product moisture. The separating layer can also be used as a light barrier by opacifying it with agents such as titanium dioxide, iron oxides and the like.

In general, the separating layer is composed of coherent or polymeric materials, and finely powdered solid excipients which constitute fillers. When a sugar is used in the separating layer, it is applied in the form of an aqueous solution and constitutes part of or the whole of the coherent material which sticks the separating layer together. In addition to or instead of the sugar, a polymeric material may also be used in the separating layer. For example, substances such as hydroxypropylmethylcellulose, polyvinylpyrrolidone, hydroxypropylcellulose and the like may be used in small amounts to increase the adherence and coherence of the separating layer.

The invention is illustrated by the following examples. The examples do not intend to limit the scope of this invention as defined in the claims below.

### EXAMPLES

### Crystallization of Duloxetine Hydrochloride form A with slow cooling (Table III)

### Example 1

Duloxetine hydrochloride form A (cf. table III for the amount) was dissolved in a selected solvent by heating to reflux temperature or suspended in a given amount of refluxing solvent to which another co-solvent was added in the amount sufficient to dissolve the salt. The resulting solution was allowed to cool to room temperature with magnetic stirring and further down to 0 - 5 °C if seemed appropriate. The solid was collected by filtration, dried in air and analyzed.

### Example 2

A mixture of duloxetine hydrochloride form A (1.0 g) was heated in a selected solvent to reflux temperature, the suspension of un-dissolved material was filtered while hot and the clear filtrate was allowed to cool to room temperature with magnetic stirring. The solid was collected by filtration, dried in air and analyzed.

### Example 3

Duloxetine hydrochloride form A (1.0 g) was dissolved in a selected solvent by warming up as necessary. Clear solution persisted after cooling down to room temperature which was allowed to stay in deep freezer for 12 days. Slow crystallization started in 1 to 6 days upon refrigeration. The solid was collected by filtration, dried in air and analyzed.

### Example 4

Duloxetine hydrochloride form A (1.0 g) was dissolved in 1,4-dioxan,e (3.5 ml) by heating to reflux temperature and the resulting solution allowed to cool to room temperature with magnetic stirring. Within 10-15 min crystallization commenced, 25-40 min later the suspension is diluted with 1,4-dioxane (5 m1), the solid was collected by filtration, dried in air and analyzed.

### Example 5

Duloxetine hydrochloride form A (1.0 g) was dissolved in 1,4-dioxane (3.5 ml) by heating to reflux temperature and the resulting solution allowed to cool to room temperature with mechanical stirring. Within 60 min crystallization commenced, 25 min later the solid was collected by filtration, dried in vacuum at room temperature for 1 day and analyzed.

### Example 6

Duloxetine hydrochloride form A (see Table III for the amount) was dissolved in 5% solution of methanol in 1,4-dioxane by heating to reflux temperature and the resulting solution allowed to cool to room temperature or to about 10 °C without any stirring. The solid was collected by filtration, dried in air and analyzed.

### Crystallization of Duloxetine Hydrochloride form A with fast cooling (Table III)

### Example 7

Duloxetine hydrochloride form A (see Table III for the amount) was dissolved in a selected solvent by heating to reflux temperature with magnetic stirring. The flask with the boiling solution was placed immediately in a cooling bath: cca. 10 °C bath for 1,4-dioxme based solvent mixtures or ice bath for the others, and the stirring was continued. The solid was collected by filtration, dried and analyzed.

### Example 8

Duloxetine hydrochloride form A (1.0 g) was dissolved in 1,4-dioxane (3.5 ml) by heating to reflux temperature with magnetic stirring. The flask with the boiling solution was placed immediately in a ~10 °C cooling bath and the stirring was continued. Within 15-30 min crystallization commenced, 15-60 min later the suspension is diluted with 1,4-dioxane (5 ml). The solid was collected by filtration, dried in air and analyzed.

### Example 9

Duloxetine hydrochloride form A (1.0 g) was dissolved in 1,4-dioxane (10 ml) by heating to reflux temperature with magnetic stirring. The flask with the boiling solution was placed immediately in a ca. 10 °C cooling bath and the stirring was continued. No crystallization took place after ca. 70 min at this temperature, therefore, the solution was allowed to warm up to ca. 20 °C. Within 20 min at 20 °C crystallization commenced, 1 h later the solid was collected by filtration, washed with fresh solvent, dried in air and analyzed.

### Example 10

Duloxetine hydrochloride form A (1.0 g) was dissolved in 1,4-dioxane (3.5 ml) by heating to reflux temperature with mechanical stirring. The flask with the boiling solution was placed immediately in a ca. 10 °C cooling bath and external stirring was continued. Within 45 min crystallization commenced, 20 min later the solid was collected by filtration, dried in vacuum at room temperature for 1 day and analyzed.

### Example 11

Duloxetine hydrochloride form A (1.0 g) was dissolved in 5% solution of methanol in 1,4-dioxane (3,5 ml) by heating to reflux temperature. The flask with the boiling solution was placed immediately in a ca. 10 °C cooling bath without any stirring applied. Crystallization commenced after ca. 7 h at this temperature. The solid was collected by filtration after 24 h, dried in air and analyzed.

### Example 12

Duloxetine hydrochloride form A (2.0 g) was dissolved in 5% solution of methanol in 1,4-dioxane (4 ml) by heating to reflux temperature with magnetic stirring. The flask with the boiling solution was placed immediately in a ca. 10 °C cooling bath and the stirring was continued. Crystallization commenced within 10 min. Wet crystalline mass was characterized directly by X-ray powder diffraction as well as after allowing the sample to dry in air for 3 days.

### Example 13:

### Crystallization of Duloxetine Hydrochloride from 2-propanol (DL-247)

Duloxetine hydrochloride form A (50.0 g) was dissolved in 2-propanol (255 ml) by heating to reflux temperature. The resulting solution was filtered while hot and the filtrate allowed to cool to room temperature with magnetic stirring for about 1 h. The solid was filtered and, while still wet, divided into three approx. equal portions, which were dried for 68 h under different conditions. First portion (19.46 g) was dried at 50 °C in vacuum to give 15.39 g of product. Second portion (19.23 g) was dried at room temperature in vacuum to give 15.74 g of product. Third portion (19.61 g) was dried in air at room temperature to give 15.17 g of product. Wet and all dried samples of duloxetine hydrochloride were shown to exist in form A according to XRPD analysis.

### Example 14:

### Crystallization of Duloxetine Hydrochloride from abs. ethanol (DL-256)

Duloxetine hydrochloride form A (50.0 g) was dissolved in abs, ethanol (50 ml) by heating to reflux temperature. The resulting solution was filtered while hot and the filtrate allowed to cool to room temperature with magnetic stirring for about 1 h. The solid was filtered and, while still wet, divided into three approx. equal portions, which were dried for 22 h under different conditions. First portion (16.86 g) was dried at 50 °C in vacuum to give 12.56 g of product. Second portion (20.17 g) was dried at room temperature in vacuum to give 17.11 g of product. Third portion (19.35 g) was dried in air at room temperature to give 15.53 g of product. Wet and all dried samples of duloxetine hydrochloride were shown to exist in form A according to XRPD analysis.

### Reference example 1 :

### Crystallization of Duloxetine Hydrochloride by slow evaporation from methanol according to WO 2005/019199A1, example 2 (DL-211)

A solution of duloxetine hydrochloride form A (1.0 g) in methanol (4 ml) was concentrated on a rotary evaporator at about 40 °C and starting pressure of 350 mbar. Within 5 hours pressure was gradually decreased to 50 mbar and evaporation continued for cca. 1 more hour to give duloxetine hydrochloride form A (1.0 g).

### Reference example 2:

### Crystallization of Duloxetine Hydrochloride from ethanol/diethyl ether mixture (DL-250)

To a refluxing solution of duloxetine hydrochloride (2.0 g) in 3 ml of ethanol was added dropwise 3.5 ml of diethyl ether. After the addition was complete, the salt crystallized out from hot solution. Additional 2 ml of ethanol was added and the resulting boiling suspension allowed to cool to room temperature with magnetic stirring. After additional 1 h at this temperature the solid was collected by filtration and dried in air to obtain duloxetine hydrochloride form A (1.81 g, 90.5%).

### Reference example 3:

### Formation of Duloxetine Hydrochloride from Duloxetine free Base according to EP0650965 (DL-257,267)

To a solution of duloxetine (4.17 g, 14.0 mmol) in 23 ml of ethyl acetate was added 1.52 g (15.4 mmol) of 37% hydrochloric acid while shaking. In few minutes the precipitation took place, after that the magnetic stirring was applied. The slurry was diluted with 23 ml of ethyl acetate, the resulting mixture was stirred for 1 hour at room temperature, and 1 hour at about 0 °C. The solid was filtered and dried under reduced pressure overnight. Duloxetine hydrochloride form A (2.89 g) was obtained according to XRPD analysis.

### Reference example 4:

### Formation of Duloxetine Hydrochloride from Duloxetine free Base according to Journal of Labeled Compounds and Radiopharmaceuticals, 36(3), 220, 1995 (DL-255)

To a solution of duloxetine (5.0 g, 16.8 mmol) in 50 ml of ethyl acetate was added dropwise 50 ml of 0.44M solution of hydrochloric acid in ethyl acetate. A white precipitate formed during addition. The slurry was stirred at room temperature for 1 h and then chilled to -20 °C for an additional hour. The mixture was diluted with 370 ml of diethyl ether and filtered. The filter cake was washed with diethyl ether (50 ml) and dried in air overnight. Duloxetine hydrochloride form A (5.62 g) was obtained according to XRPD analysis.

1,00 g of this product was recrystallized as follows. To a refluxing solution in 4 ml of ethanol was added dropwise 4 ml of diethyl ether. After the addition was complete, the salt crystallized out from hot solution. The resulting suspension was allowed to cool to room temperature with magnetic stirring. After additional 10 min at this temperature the solid was collected by filtration, washed twice with 5 ml of diethyl ether and dried in air. Form A of the recrystallized product (0.88 g) was obtained according to XRPD analysis.

### Reference example 5:

### Formation of Duloxetine Hydrochloride from Duloxetine free Base according to WO2005/198386 (DL-270)

To a solution of duloxetine (4.6 g, 15.5 mmol) in 46 ml of acetone was added 2.9 g (16 mmol) of 20% hydrochloric acid in 2-propanol while shaking, after that the magnetic stirring was applied. In 30 minutes the precipitation took place and the resulting mixture was stirred for 2 hours at room temperature. The solid was filtered, washed with 18 ml of acetone and dried under reduced pressure at 50-55 °C. Duloxetine hydrochloride form A (2.97 g) was obtained according to XRPD analysis.

### Reference example 6:

### Synthesis of Duloxetine Hydrochloride (Reaction conditions according to »Preparation 2« of EP0650965B1)(DL-245)

To a stirred mixture of (*S*)-(+)-*N,N*-dimethyl-3-(1-naphthalenyloxy-3-(2-thienyl)propan-amine oxalate (4.90 g, 12.2 mmol) in 40 ml of toluene and 40 ml of water was added 5 ml of 30% ammonium hydroxide solution at 40 °C. Stirring was continued for 10 min at this temperature and the layers were separated. The organic phase was washed with water, dried with magnesium sulfate and filtered. The filtrate was concentrated to about half volume and heated to 55 °C. Then 0.16 g of diisopropylethylamine was added, followed by the dropwise addition of 2.39 g (15.26 mmol) of phenyl chloroformate. The mixture was stirred at 55 °C for 75 min, then 50 ml of 1% sodium bicarbonate solution was added. After stirring at 40-50 °C for 10 min the phases were separated and the organic phase was washed with 0.5 N hydrochloric acid, 1% sodium bicarbonate solution and concentrated. The residue was dissolved in 52 ml of dimethylsuffoxide, the solution was heated to 45 °C, and a solution of 2 g of sodium hydroxide in 12 ml of water was added dropwise. The mixture was stirred at 50 °C for 19 hours, then cooled to room temperature, diluted with 34 ml of water, and acidified to pH 5.4 by addition of acetic acid. Then 40 ml of hexane was added, the mixture was stirred for 10 min, the phases were separated and the treatment with hexane was repeated once more. The resulting aqueous phase was made basic to pH 10.5 with 30% aqueous sodium hydroxide and 34 ml of ethyl acetate was added. After stirring for 10 min, the phases were separated and the aqueous phase was extracted with another 34 ml portion of ethyl acetate. The combined organic extracts were washed with water and concentrated to 20 ml under vacuum. To this solution was added 0.92 g of concentrated hydrochloric acid, then a small amount of crystalline duloxetine hydrochloride (pure polymorph A) and an additional 20 ml of ethyl acetate. The mixture was stirred for about 1 hour more and concentrated to 20 ml under vacuum. The solution was stirred for 1 h at room temperature, then for 1 h at about 0 °C, and finally kept in the refrigerator overnight. Since no precipitation took place, the solution was concentrated to an oily residue, which showed no tendency of crystallization within a period of one week. This was redissolved in 35 ml of ethyl acetate then 170 ml of diethyl ether was added dropwise. The mixture was stirred for 1 h, the solid was filtered and washed with diethyl ether to obtain 1.22 g of duloxetine hydrochloride (polymorph A).

### Formulation example 1: Pharmaceutical formulation of Duloxetine hydrochloride

### Example A

| **Ingredients** | **Composition** |
|---|---|
| **Core material** | |
| Sucrose-starch nonpareils, 30-35 mesh | 85,69 mg |
| Duloxetine hydrochloride | 44,84 mg |
| Hydroxypropyl methylcellulose | 5,32 mg |
| | |

| **Separating layer** | |
|---|---|
| Hydroxypropyl methylcellulose | 4,07 mg |
| Talc | 17,26 mg |
| Sucrose | 3,61 mg |
| | |

| **Enteric layer** | |
|---|---|
| methacrylic acid ethyl acrylate copolymer | 32,16 mg |
| Triethyl citrate | 3,22 mg |
| Talc | 16,08 mg |
| | |

| **Finishing layer** | |
|---|---|
| Hydroxypropyl methylcellulose | 7,75mg |
| Titanium dioxide | 10,00mg |
| **Total** | **230 mg** |

The duloxetine layering was performed in a fluid bed processor using bottom spray technique (Wurster column) at a batch size of 1.5 kg. The duloxetine (average particle size 14 µm) was suspended in the hydroxypropyl methylcellulose water solution. The suspension was slowly sprayed onto the agitating batch of beads. The prepared core material was dried until the loss on drying of the pellets was below 0.5 % and covered with separating layer in a Wurster column with a water solution of hydroxypropyl methylcellulose containing talc and sucrose.

The enteric coating suspension was prepared using methacrylic acid ethyl acrylate copolymer (Eudragit L 30 D-55), triethyl citrate and talc. The suspension was sprayed onto the pellets covered with separating layer in a fluid bed apparatus. The spray operation was stopped when the specified amount of bulk liquid had been sprayed, and then drying was carried in the Wurster column.

The final coating was applied in the Wurster column by spraying the dispersion of hydroxypropyl methylcellulose and titanium dioxide on the enteric-coated pellets. Drying in the same apparatus followed the procedure. The pellets were filled into #2 gelatine capsules.

### Example B:

| **Ingredients** | **Composition** |
|---|---|
| **Core material** | |
| Microcrystalline cellulose beads, 30-35 mesh | 137,10 mg |
| Duloxetine hydrochloride | 67,36 mg |
| Hydroxypropyl methylcellulose | 7,33 mg |
| | |

| **Separating layer** | |
|---|---|
| Hydroxypropyl methytceHulose | 9,54 mg |
| Titanium dioxide | 2,23 mg |
| Magnesium stearate | 5,25 mg |
| | |

| **Enteric layer** | |
|---|---|
| Hydroxypropyl methylcellulose phthalate | 40,25 mg |
| Diethyl phthalate | 4,40 mg |
| Talc | 7,69 mg |
| Titanium dioxide | 1,75 mg |
| | |

| **Finishing layer** | |
|---|---|
| Hydroxypropyl methylcellulose | 12,25 mg |
| Talc | 0,49 mg |
| Titanium dioxide | 4,36 mg |
| **Total** | **300 mg** |

Drug suspension was prepared using duloxetine (average particle size 14 µm), hydroxypropyl methylcellulose and purified water. Suspension layering of microcrystalline cellulose beads was performed in a Wurster column at a batch size of 2,0 kg. In the same apparatus the prepared core material was covered with a hydroxypropyl methylcellulose solution containing titanium dioxide and magnesium stearate. The enteric coating suspension was then prepared using hydroxypropyl methylcellulose phthalate, diethyl phthalate, talc, titanium dioxide, methanol and methylene chloride. The said suspension was sprayed onto the pellets in the Wurster column. Additional coating was applied by spraying the dispersion of hydroxypropyl methylcellulose and titanium dioxide on the enteric-coated pellets. The pellets were filled into #1 gelatine capsules.

### Example C:

| **Ingredients** | **Composition** |
|---|---|
| **Core material** | |
| Sucrose-starch nonpareils, 25-30 mesh | 106,24 mg |
| Duloxetine hydrochloride | 22,45 mg |
| Hydroxypropyl methylcellulose | 4,12 mg |
| | |

| **Separating layer** | |
|---|---|
| Hydroxypropyl methylcellulose | 3,07 mg |
| | |

| **Enteric layer** | |
|---|---|
| Hydroxypropyl methylcellulose acetate succinate | 22,63 mg |
| Dibutyl sebacate | 2,17 mg |
| Talc | 9,32 mg |
| **Total** | **170 mg** |

The product was made in the Wurster apparatus at a batch size of 1.0 kg. The duloxetine layer was built up by spraying a suspension of duloxetine in an aqueous solution of hydroxypropyl methylcellulose. The separating layer was applied by spraying an aqueous solution of hydroxypropyl methylcellulose. The enteric polymer was neutralized with ammonium hydroxide to dissolve it in water at pH 6.9. Dibutyl sebacate and talc were added to the solution, while gently stirring. The enteric coating suspension was applied to the subcoated granules in the Wurster column at inlet air temperature of about 70 °C. After drying the pellets were filled into size #3 gelatine capsules.

| | | | | | | | |
|---|---|---|---|---|---|---|---|
| DL-215 | 2 | 1.0 | 2-Butanone | 38 | 79.5 | *t*_{CP} : 16 h at room temp., *t*_{BC}: | A |
| DL-216 | 3 | 1.0 | Methanol | 3 | 53 | during cooling *t*_{CP} : 12 days at -20 °C, *t*_{BC:} cca. 3 | A |
| DL-217 | 3 | 1.0 | 2-Methoxyethanol | 3 | 20 | days *t*_{CP} : 12 days at -20 °C, *t*_{BC}: cca. 6 days | A |
| OL-218 | 3 | 1.0 | Water | 3 | 46 | *t*_{CP} : 12 days at -20 °C. *t*_{BC}: cca. 1 day | A |
| DE-221A | 4 | 1.0 | 1.4-Dioxane | 3.5 + 5 | 72 | *t*_{CP} : 55 min, *t*_{BC}: 15 min | T >A |
| DL-222B | 4 | 1.0 | 1,4-Dioxane | 3.5 + 5 | 68 | *t*_{CP} : 35 min, *t*_{BC}: 10 min | A |
| DL-242 | 5 | 1.0 | 1,4-Dioxane | 3.5 | 47 | *t*_{CP} : 85 min, *t*_{BC}: 60 min; mechanical stirring | A+T |
| DL-244A | 6 | 1.0 | 5% methanol in 1,4- dioxane | 3.5 | 78 | *t*_{CP} : 24 h, *t*_{BC}: cca. 7 h, without any stirring | T |
| DL-268 | 6 | 5.0 | 5% methanol in 1,4- dioxane | 17.5 | 78 | *t*_{CP} : 2 h at room temp., 20 h at cca. 10 °C; without any stirring | T |
| DL-269 | 6 | 5.0 | 5% methanol in 1,4- dioxane | 97.5 | 76 | *t*_{CP} : 2 h at room terrsp., 20 h et cca. 10 °C; without any stirring | T |
| DL-226 | 7 | 1.0 | Water | 3.5 | 43 | *t*_{CP} : 22 h, cca. 0.5 h at 0 °C, the rest at room temp., *t*_{BC}: 15 min | A |
| DL-2248 | 7 | 1.0 | Ethanol abs. | 3.5 | 23 | *t*_{CP} : 30 min, *t*_{BC}: during cooling | A |
| DL-224A | 7 | 1.0 | Acetanitrile | 10 | 85 | *t*_{CP} : 35 min, *t*_{BC}: during cooling | A |
| DL-223A | 7 | 1.0 | 1,4-Dioxane | 3.5 | 46 | *t*_{CP} : cca. 1 h, *t*_{BC}: 10 min | A |
| DL-227 | 7 | 1.0 | 98% aq. 1,4-Dioxane | 3 | 47 | *t*_{CP} : cca. 2.5 h, *t*_{BC}: cca. 1.5 h | T >A |
| DL-228 | 7 | 1.0 | 95% aq. 1,4-Dioxane | 3 | - | No crystallization at cca. 10 °C or room temp. | - |
| DL-229 | 7 | 1.0 | 90% aq. 1,4-Dioxane | 2 | - | No crystallization at cca. 10 °C or room temp. | - |
| DL-232A | 7 | 1.0 | 2% 2-Methoxyethanol in 1,4-dioxane | 2 | 62 | *t*_{CP}: 40 min, *t*_{BC}: cca. 10 min | T >A |
| DL-232B | 7 | 1.0 | 5% 2-Methoxyethanol in 1,4-dioxane | 2 | 59 | *t*_{CP}: 95 min, *t*_{BC}: cca. 15 min | T >A |
| DL-233 | 7 | 1.0 | 10% 2-Methoxyethanol in 1,4-rtioxane | 2 | 66 | *t*_{CP}: 40 min, *t*_{BC}: cca. 10 min | A |
| DL-230A | 7 | 1.0 | 2% Methanol in 1,4- dioxane | 2 | 73.5 | *t*_{CP}: 40 min, *t*_{BC}: cca. 10 min | T >>A |
| DL-239B | 7 | 1.0 | 2% Methanol in 1,4- dioxane | 2 | 93.5 | *t*_{CP}: 45 min, *t*_{BC}: 15 min | A+T |
| DL-230B | 7 | 1.0 | 5% Methanol in 1,4- dioxane | 2 | 44.5 | *t*_{CP}: 40 min, *t*_{BC}: cca. 10 min | T ^{b} |
| DL-234A | 7 | 1.0 | 5% Methanol in 1,4- dioxane | 2 | 73 | *t*_{CP}: 35 min, *t*_{BC}: cca. 15 min | T >A |
| DL-234B | 7 | 1.0 | 5% Methanol in 1,4- dioxane | 2 | 73 | *t*_{CP}: 60 min, t_{BC:} cca. 10 min | T >A |
| DL-234C | 7 | 1.0 | 5% Methanol in 1,4- dioxane | 2 | 80 | *t*_{CP}: 30 min, *t*_{BC}: 7 min | T A |
| DL-239A | 7 | 1.0 | 5% Methanol in 1,4- dioxane | 2 | 85 | *t*_{CP}: 18 min. *t*_{BC}**:** 8 min | T ^{c} |
| DL-235A | 7 | 1.0 | 5% Methanol in 1,4- dioxane | 3 | 75 | *t*_{CP}: 70 min, *t*_{BC}: 15 min | T >A |
| DL-2358 | 7 | 1.0 | 5% Methanol in 1,4- dioxane | 4 | 56 | *t*_{CP}: 45 min, *t*_{BC}: 25 min | A |
| DL-235C | 7 | 1.0 | 5% Methanol in 1,4- dinxane | 5 | 64 | *t*_{CP}: 50 min, *t*_{BC}: 25 min | A + T |
| DL-231 | 7 | 1.0 | 10% Methanol in 1,4- dioxane | 2 | 44.5 | *t*_{CP}: 45 min, *t*_{BC}: cca. 15 min | T A |
| DL-221B | 8 | 1.0 | 1,4-dioxane | 3.5 + 5 | 72 | *t*_{CP}: 55 min, *t*_{BC}: 40 min | T A |
| DL-222A | 8 | 1.0 | 1,4-dioxane | 3.5 + 5 | 59 | *t*_{CP}: cca. 1.5 h, *t*_{BC}: 30 min | A + T |
| DL-222C | 9 | 1.0 | 1,4-dioxans | 10 | 66.5 | See. Example 9 | T >A |
| DL-243 | 10 | 1.0 | 1,4-dioxane | 3.5 | 75 | *t*_{CP}: 65 min, *t*_{BC}: 45 min; mechanical stirring | A + T |
| DL-244B | 11 | 1.0 | 5% Methanol in 1,4- dioxane | 3.5 | 82 | *t*_{CP}: 24 h, *t*_{BC}: cca. 7 h; without any stirring | A + T ^{d} |
| DL-238 | 12 | 2.0 | 5% Methanol in 1,4- dioxane | 4 | - | *t*_{BC}: 10 min; characterization of wet and, after 3 d, air- dried material | T (wet) A + T (after 3 d) |
| DL-235B | 7 | 1.0 | dioxane 5% Methanol in 1,4- dioxane | 4 | 56 | *t*_{CP}: 45 min, *t*_{BC}: 25 min | A |
| DL-235C | 7 | 10 | 5% Methanol in 1,4- dioxane | 5 | 64 | *t*_{CP}: 50 min, *t*_{BC}: 25 min | A + T |
| DL-231 | 7 | 1.0 | 10% Methanol in 1,4- dioxane | 2 | 44.5 | *t*_{CP}: 45 min, *t*_{BC}: cca. 15 min | T >A |
| DL-221B | 8 | 1.0 | 1,4-dioxane | 3.5 + 5 | 72 | *t*_{CP}: 55 min, *t*_{BC}: 40 min | T A |
| DL-222A | 8 | 1.0 | 1,4-dioxane | 3.5 + 5 | 59 | *t*_{CP}: cca. 1.5 h, *t*_{BC}: 30 min | A + T |
| DL-222C | 9 | 1.0 | 1,4-dioxane | 10 | 66.5 | See: Example 9 | |
| DL-243 | 10 | 1.0 | 1,4-dioxane | 3.5 | 75 | *t*_{CP}: 65 min, *t*_{BC}: 45 min; mechanical stirring | T >A A + T |
| DL-244B | 11 | 1.0 | 5% Methanol in 1,4- dioxane | 3.5 | 82 | *t*_{CP}: 24 h, *t*_{BC}. cca. 7 h, without any stirring | A + T ^{d} |
| DL-238 | 12 | 2.0 | 5% Methanol in 1,4- dioxane | 4 | - | *t*_{BC}: 10 min; characterization of wet and, after 3 d, air- dried material | T (wet) A + T (after 3 d) |

| | | | | | | | |
|---|---|---|---|---|---|---|---|
| ^{a} *t*_{CP}; time of the crystallization process: since the start of cooling until the collection of crystals; *t*_{BC}; time before the crystal formation takes place ^{b} Stable sample: XRPD unchanged after 22 days ^{c} Stable sample: XRPD unchanged after 12 days ^{d} Fraction of form T decreases with time in favor of form A. ^{a} *t*_{CP}; time of the crystallization process: since the start of cooling until the collection of crystals; *t*_{BC}; time before the crystal formation takes place ^{b} Stable sample: XRPD unchanged after 22 days ^{c} Stable sample: XRPD unchanged after 12 days ^{d} Fraction of form T decreases with time in favor of form A. | | | | | | | |

The present invention pertains to the following special embodiments:
Item 1. A process for the preparation of crystalline duloxetine HCl characterized by an X-ray diffraction pattern with peaks (2θ):9.6, 13.9, 18.0, 18.8, 19.2, 20.8, 27.4, 27.9 (designated form A), which comprises the steps of
   (i) dissolving duloxetine HCl in a solvent mixture comprising 1,4-dioxane;
   (ii) optionally heating the solution;
   (iii) cooling the solution to a temperature of from about - 20 °C to about 30 °C to induce crystallization;
   (iv) recovering the crystals thus formed; and optionally
   (v) drying the crystals thus obtained.
Item 2. The process according to item 1, wherein the solvent contains alcohols.
Item 3. The process according to any of the items 2 or 3, wherein the solution is cooled in step (iii) to a temperature of from about - 10 °C to about 20 °C, preferably 5 °C to about 10 °C.

## Claims

1. A pharmaceutical formulation of duloxetine hydrochloride comprising
a) a core material comprising duloxetine hydrochloride,
b) an optional separating layer, and
c) one or more enteric layers applied by coating an enteric coating layer material onto the core material or onto the core material covered with the separating layer, wherein the enteric coating layer material comprises one or more enteric coating layer polymers selected from the group consisting of solutions or dispersions of methacrylic acid copolymers, cellulose acetate phthalate, hydroxypropyl methylcellulose phthalate, hydroxypropyl methylcellulose acetate succinate, polyvinyl acetate phthalate, cellulose acetate trimellitate, carboxymethylethyl-cellulose.

2. The pharmaceutical formulation of duloxetine hydrochloride according to claim 1, wherein the one or more enteric coating layer polymers are selected from the group consisting of solutions or dispersions of methacrylic acid copolymers, cellulose acetate phthalate, hydroxypropyl methylcellulose phthalate, polyvinyl acetate phthalate, cellulose acetate trimellitate, carboxymethylethyl-cellulose.

3. The pharmaceutical formulation of duloxetine hydrochloride according to any of the preceding claims, wherein the one or more enteric coating layer polymers are selected from the group consisting of solutions or dispersions of methacrylic acid copolymers and hydroxypropyl methylcellulose phthalate.

4. The pharmaceutical formulation of duloxetine hydrochloride according to any of the preceding claims, wherein the separating layer comprises sugar and/or a water soluble polymer selected from the group consisting of hydroxypropylmethylcellulose, polyvinylpyrrolidone, hydroxypropylcellulose.

5. The pharmaceutical formulation of duloxetine hydrochloride according to any of the preceding claims wherein the separating layer comprises hydroxypropyl methylcellulose.

6. The pharmaceutical formulation of duloxetine hydrochloride according to any of the preceding claims, wherein one or more enteric layer further comprises one or more plasticizers.

7. The pharmaceutical formulation of duloxetine hydrochloride according to claim 6, wherein the one or more plasticizers are selected from the group consisting of triacetin, phthalic acid esters, dibutyl sebacate, dimethyl polysiloxan, cetyl alcohol, stearoyl alcohol, polyethylene glycols, and propylenglycol,

8. The pharmaceutical formulation of duloxetine hydrochloride according to claims 6 or 7 wherein the one or more plasticizers are selected from diethyl phthalate or polyethylene glycol.

9. The pharmaceutical formulation of duloxetine hydrochloride according to any of the preceding claims, wherein the dose of duloxetine is 1-50 mg.

10. The pharmaceutical formulation of duloxetine hydrochloride according to any of the preceding claims, wherein the particle size of duloxetine hydrochloride is less than 120 µm.

11. The pharmaceutical formulation of duloxetine hydrochloride according to any of the preceding claims, wherein the formulation is in the form of pellets having a size of from 0.1 to 2 mm, preferably 0.5 to 1.5 mm.

12. The pharmaceutical formulation of duloxetine hydrochloride according to any of the preceding claims, wherein the core material is an inert bead onto which a duloxetine-containing layer is applied.

13. The pharmaceutical formulation of duloxetine hydrochloride according to claim 12, wherein the inert bead has a size of from 0.3 to 0.8 mm.

14. Process for manufacturing the pharmaceutical formulation according to any one of the preceding claims, comprising
the step of providing a core material comprising duloxetine hydrochloride, wherein the core material is optionally covered with a separating layer, and
the step wherein an enteric coating layer material comprising one or more enteric coating layer polymers selected from the group consisting of solutions or dispersions of methacrylic acid copolymers, cellulose acetate phthalate, hydroxypropyl methylcellulose phthalate, hydroxypropyl methylcellulose acetate succinate, polyvinyl acetate phthalate, cellulose acetate trimellitate, carboxymethylethyl-cellulose is applied by coating onto said core material or onto said core material covered with separating layer.
